(19) Europäisches Patentamt
European Patent Office
Office européen des brevets

(11) **EP 2 221 653 A1**

(12) **EUROPÄISCHE PATENTANMELDUNG**

(43) Veröffentlichungstag:
**25.08.2010 Patentblatt 2010/34**

(51) Int Cl.:
**G02B 21/06** *(2006.01)*  **A61B 19/00** *(2006.01)*
**G02B 21/00** *(2006.01)*  **A61B 5/00** *(2006.01)*
**G01B 9/02** *(2006.01)*

(21) Anmeldenummer: **10155318.8**

(22) Anmeldetag: **10.10.2007**

(84) Benannte Vertragsstaaten:
**DE ES FR GB IT**

(30) Priorität: **06.11.2006 DE 102006052513**
**24.04.2007 DE 102007019677**

(62) Dokumentnummer(n) der früheren Anmeldung(en) nach Art. 76 EPÜ:
**07019793.4 / 1 918 756**

(71) Anmelder: **Carl Zeiss Surgical GmbH**
**73447 Oberkochen (DE)**

(72) Erfinder:
• **Reimer, Peter**
  **73479, Ellwangen (DE)**
• **Hauger, Christoph**
  **73431, Aalen (DE)**
• **Abele, Alfons**
  **73527, Schwäbisch Gmünd (DE)**
• **Seeßelberg, Markus**
  **73431, Aalen (DE)**

Bemerkungen:
Diese Anmeldung ist am 03-03-2010 als Teilanmeldung zu der unter INID-Code 62 erwähnten Anmeldung eingereicht worden.

(54) **Operationsmikroskop mit OCT-System**

(57) Die Erfindung betrifft ein Operationsmikroskop 100 mit einem Beobachtungsstrahlengang 105 zur Untersuchung eines Objektbereichs 108 und mit einem Objektiv 101, das von dem Beobachtungsstrahlengang 105 des Operationsmikroskops 100 durchsetzt wird. Das Operationsmikroskop 100 umfasst ferner ein OCT-System 152 zur Untersuchung des Objektbereichs mit einem OCT-Abtaststrahlengang 153, der durch das Objektiv 101 geführt ist, einem Lichtleiter 151, der einen Lichtaustrittsabschnitt für den OCT-Abtaststrahlengang 153 aufweist und einer Kollimationsoptik 157, welche den OCT-Abtaststrahlengang 153 mit einer Linsengruppe 126 in einen im wesentlichen parallelen Abtaststrahlengang überführt.

Erfindungsgemäß sind zum Einstellen einer OCT-Abtastebene 170 Mittel zum Bewegen 171, 174 vorgesehen, mit denen sich der Lichtaustrittsabschnitt 174 des Lichtleiters 151 und/oder die Kollimationsoptik 157 bewegen lässt.

FIG.1

EP 2 221 653 A1

**Beschreibung**

Die Erfindung betrifft ein Operationsmikroskop mit einem Beobachtungsstrahlengang zur Untersuchung eines Objektbereichs, mit einem Objektiv, das von dem Beobachtungsstrahlengang des Operationsmikroskops (100) durchsetzt wird und mit einem OCT-System zur Untersuchung des Objektbereichs, wobei das OCT-System einen OCT-Abtaststrahlengang, der durch das Objektiv (101) geführt ist, einen Lichtleiter, der einen Lichtaustrittsabschnitt für den OCT-Abtaststrahlengang aufweist, und eine Kollimationsoptik umfasst, welche den OCT-Abtaststrahlengang mit einer Linsengruppe in einen im wesentlichen parallelen Abtaststrahlengang überführt, umfasst.

mit einer Beleuchtungseinrichtung, die eine Beleuchtungsoptik umfasst, welche eine von einer Lichtquelle ausgeleuchtete Leuchtfeldblende zu einem parallelen Beleuchtungsstrahlengang nach unendlich abbildet, wobei die Beleuchtungsoptik eine erste Linsengruppe und eine zweite Linsengruppe aufweist, mit einem Objektiv, das im parallelen Beleuchtungsstrahlengang angeordnet ist, um die Leuchtfeldblende auf den Objektbereich abzubilden, und mit einem OCT-System zur Untersuchung des Objektbereichs, wobei das OCT-System einen OCT-Abtaststrahlengang umfasst, der durch das Objektiv geführt ist.

Die Erfindung betrifft weiter ein Operationsmikroskop-Beleuchtungsmodul zum Anschluss an ein Operationsmikroskop mit einer Aufnahme für einen ersten Lichtleiter, um Beleuchtungslicht bereitzustellen, mit einer Leuchtfeldblende, die mit Licht aus dem Lichtleiter ausgeleuchtet werden kann, mit einer Beleuchtungsoptik, welche die Leuchtfeldblende zu einem parallelen Abbildungsstrahlengang nach unendlich abbildet, wobei die Beleuchtungsoptik eine erste Linsengruppe und eine zweite Linsengruppe aufweist und mit einem Beleuchtungsspiegel, der dazu dient, das aus der Leuchtfeldblende austretende Beleuchtungslicht mit parallelem Abbildungsstrahlengang durch das Mikroskop-Hauptobjektiv zum Objektbereich zu lenken.

Ein Operationsmikroskop der eingangs genannten Art ist aus der EP 0 815 801 bekannt.

Dieses Operationsmikroskop enthält ein OCT-System, das einen OCT-Abtaststrahlengang aus kurzkohärenter Laserstrahlung erzeugt. Das OCT-System enthält eine Analyseeinheit zur Auswertung von Interferenzsignalen. Es umfasst eine Einrichtung zum Scannen des OCT-Abtaststrahlengangs mit zwei Scanspiegeln, die um zwei Bewegungsachsen verstellt werden können. Der OCT-Abtaststrahlengang in dem Operationsmikroskop ist über einen Teilerspiegel in den Beleuchtungsstrahlengang des Operationsmikroskops eingekoppelt. Er wird mit diesem durch das Mikroskop-Hauptobjektiv hindurch zum Objektbereich gelenkt.

Ein Operationsmikroskop-Beleuchtungsmodul der eingangs genannten Art enthält das Carl Zeiss Operationsmikroskopsystem OPMI® Visu 200. Dieses Beleuchtungsmodul ist zur Befestigung an dem Grundkörper eines Operationsmikroskops ausgebildet. Es umfasst als Beleuchtungsoptik zwei Linsengruppen, die eine mit Licht aus dem Lichtleiter ausgeleuchtete Leuchtfeldblende in einen parallelen Abbildungsstrahlengang überführen, der senkrecht zur optischen Achse des Mikroskop-Hauptobjektivs verläuft, wenn das Beleuchtungsmodul an das Operationsmikroskop angeschlossen ist. Das Beleuchtungsmodul enthält zwei Beleuchtungsspiegel, die das Beleuchtungslicht parallel zur optischen Achse des Mikroskop-Hauptobjektivs umlenken.

Ein OCT-System erlaubt mittels optischer Kohärenztomographie die nichtinvasive Darstellung und Messung von Strukturen innerhalb eines Gewebes. Als optisches bildgebendes Verfahren ermöglicht die optische Kohärenztomographie insbesondere Schnitt- oder Volumenbilder von biologischem Gewebe mit Mikrometerauflösung zu erzeugen. Ein entsprechendes OCT-System umfasst eine Quelle für zeitlich inkohärentes und räumlich kohärentes Licht mit einer Kohärenzlänge $l_c$, die einem Probenstrahlengang und einem Referenzstrahlengang zugeführt wird. Der Probenstrahlengang ist auf das zu untersuchende Gewebe gerichtet. Laserstrahlung, die aufgrund von Streuzentren im Gewebe in den Probenstrahlengang zurückgestrahlt wird, überlagert das OCT-System mit Laserstrahlung aus dem Referenzstrahlengang. Durch die Überlagerung entsteht ein Interferenzsignal. Aus diesem Interferenzsignal lässt sich die Position von Streuzentren für die Laserstrahlung im untersuchten Gewebe bestimmen.

Für OCT-Systeme ist das Bauprinzip des "Time-Domain OCT" und des "Fourier-Domain OCT" bekannt.

Der Aufbau eines "Time-Domain OCT" ist beispielsweise in der US 5,321,501 anhand von Fig. 1a auf Sp. 5, Z. 40 - Sp. 11, Z. 10 beschrieben. In einem solchen System wird die optische Weglänge des Referenzstrahlenganges über einen schnell beweglichen Referenzspiegel fortlaufend variiert. Das Licht aus Proben- und Referenzstrahlengang wird auf einem Photodetektor überlagert. Wenn die optischen Weglängen von Proben- und Referenzstrahlengang übereinstimmen, entsteht auf dem Photodetektor ein Interferenzsignal.

Ein "Fourier-Domain OCT" ist beispielsweise in der WO 2006/10544 A1 erläutert. Um die optische Weglänge eines Probenstrahlenganges zu vermessen, wird wiederum Licht aus dem Probenstrahlengang Licht aus einem Referenzstrahlengang überlagert. Im Unterschied zu einem "Time-Domain OCT" wird jedoch für eine Messung der optischen Weglänge des Probenstrahlenganges das Licht aus Proben- und Referenzstrahlengang nicht direkt einem Detektor zugeführt, sondern zunächst mittels eines Spektrometers spektral zerlegt. Die so erzeugte spektrale Intensität des überlagerten Signals aus Proben- und Referenzstrahlengang wird dann mit einem Detektor erfasst. Durch Auswerten des Detektorsignals kann wiederum die optische Weglänge des Probenstrahlenganges ermittelt werden.

Aufgabe der Erfindung ist es, ein Operationsmikroskop mit OCT-System zu schaffen, bei dem eine OCT-Abtastebene unabhängig von einer Beobachtungsebene eines optischen Beobachtungsstrahlengangs einstellbar ist. das ein geringes Bauvolumen hat und dessen Bauprinzip ein einfaches Nachrüsten von Operationsmikroskopen für OCT ermöglicht, sowie ein Operationsmikroskop-Beleuchtungsmodul mit integriertem OCT-System zum Anschluss an ein Operationsmikroskop bereitzustellen.

Die Aufgabe wird durch ein Operationsmikroskop gelöst, bei dem zum Einstellen einer OCT-Abtastebene Mittel zum Bewegen vorgesehen sind, mit denen sich der Lichtaustrittsabschnitt des Lichtleiters und/oder die Kollimationsoptik bewegen lässt.

Diese Aufgabe wird durch ein Operationsmikroskop der eingangs genannten Art gelöst, bei dem im Beleuchtungsstrahlengang zwischen der ersten Linsengruppe und der zweiten Linsengruppe ein Einkoppelelement vorgesehen ist, das den OCT-Abtaststrahlengang in den Beleuchtungsstrahlengang einkoppelt, sowie ein Operationsmikroskop-Beleuchtungsmodul der eingangs genannten Art gelöst, bei dem eine Aufnahme für einen zweiten Lichtleiter eines OCT-Systems vorgesehen ist, um einen OCT-Abtaststrahlengang bereitzustellen, wobei das Operationsmikroskop-Beleuchtungsmodul ein zwischen der ersten Linsengruppe und der zweiten Linsengruppe angeordnetes Einkoppelelement umfasst, das den OCT-Abtaststrahlengang in den Beleuchtungsstrahlengang einkoppelt.

In Weiterbildung der Erfindung ist bei dem Operationsmikroskop bzw. dem Operationsmikroskop-Beleuchtungsmodul das Einkoppelelement als Teilerspiegel, insbesondere als Planspiegel oder Teilerwürfel ausgebildet.

In Weiterbildung der Erfindung ist bei dem Operationsmikroskop das im parallelen Beleuchtungsstrahlengang angeordnete Objektiv als Mikroskop-Hauptobjektiv ausgebildet und wird von dem Beobachtungsstrahlengang des Operationsmikroskops durchsetzt. Auf diese Weise wird eine besonders kompakte Bauform des Operationsmikroskops erzielt.

In Weiterbildung des Operationsmikroskops ist auf der dem Objekt abgewandten Seite des Objektivs eine Umlenkeinrichtung für Beleuchtungslicht vorgesehen, die das Beleuchtungslicht der Beleuchtungseinrichtung zum Objektiv hin umlenkt. Auf diese Weise ist eine zu den Beobachtungsstrahlengängen achsnahe Beleuchtung möglich, die für ophthalmologische Operationen von Vorteil sein kann.

In Weiterbildung der Erfindung ist die Umlenkeinrichtung als Strahlenteiler ausgebildet, der von dem Beobachtungsstrahlengang des Operationsmikroskops durchsetzt ist. Auf diese Weise kann Beleuchtungslicht in den Beobachtungsstrahlengängen des Operationsmikroskops zum Objektbereich geführt werden.

In Weiterbildung der Erfindung enthält das Operationsmikroskop ein OCT-System für das Scannen des OCT-Abtaststrahlengangs mit einem ersten Scanspiegel. Vorzugsweise ist zusätzlich ein zweiter Scanspiegel vorgesehen, wobei der erste Scanspiegel um eine erste Drehachse bewegt werden kann und sich der zweite Scanspiegel um eine zweite Drehachse bewegen lässt, und wobei die erste Drehachse und die zweite Drehachse seitlich versetzt in einem rechten Winkel zueinander stehen. Auf diese Weise ist ein Abscannen des Objektbereichs mit senkrecht verlaufendem Rastermuster möglich.

In Weiterbildung der Erfindung umfasst das Operationsmikroskop ein OCT-System mit einem Lichtleiter der einen Lichtaustrittsabschnitt für den OCT-Abtaststrahlengang hat, der bewegbar gehalten ist. Auf diese Weise kann eine OCT-Abtastebene im Objektbereich variiert werden und es ist möglich, das System für unterschiedliche OCT-Wellenlängen in anbetracht der für sichtbares Licht ausgelegten optischen Komponenten im Beobachtungsstrahlengang einzustellen.

In Weiterbildung der Erfindung umfasst das Operationsmikroskop ein OCT-System mit verstellbarer Kollimationsoptik, welche den OCT-Abtaststrahlengang mit der zweiten Linsengruppe der Beleuchtungsoptik in einen im wesentlichen parallelen Abtaststrahlengang überführt. Auf diese Weise ist es möglich, die OCT-Abtastebene bei dem Operationsmikroskop relativ zur Beobachtungsebene der optischen Beobachtungsstrahlengänge des Systems zu verlagern.

Vorzugsweise umfasst das Operationsmikroskop-Beleuchtungsmodul eine Einrichtung zum Scannen des OCT-Abtaststrahlengangs. Diese Einrichtung zum Scannen kann beispielsweise einen ersten Scanspiegel und einen zweiten Scanspiegel aufweisen. Indem der erste Scanspiegel um eine erste Drehachse bewegt werden kann und der zweite Scanspiegel sich um eine zweite Drehachse verstellen lässt, wobei die erste Drehachse und die zweite Drehachse seitlich versetzt in einem rechten Winkel zueinander stehen, kann ein Objektbereich mit senkrecht verlaufendem Rastermuster abgescannt werden.

In Weiterbildung der Erfindung ist der Lichtaustrittsabschnitt des Lichtleiters für den OCT-Abtaststrahlengang im Operationsmikroskop-Beleuchtungsmodul bewegbar gehalten, um so die OCT-Abtastebene einstellen zu können.

Indem im Operationsmikroskop-Beleuchtungsmodul eine verstellbare Kollimationsoptik vorgesehen ist, mittels derer der OCT-Abtaststrahlengang mit der zweiten Linsengruppe der Beleuchtungsoptik in einen im wesentlichen parallelen Abtaststrahlengang überführt werden kann, ist es möglich, die OCT-Abtastebene des OCT-Systems relativ zu einer Beobachtungsebene für optische Beobachtungsstrahlengänge zu verlagern.

Vorteilhafte Ausführungsformen der Erfindung sind in den Figuren dargestellt und werden nachfolgend beschrieben.

Es zeigen:

Fig. 1 ein Operationsmikroskop mit Beleuchtungsmodul, in das ein OCT-System integriert ist;

Fig. 2 einen Schnitt des Mikroskop-Hauptobjektivs entlang der Linie II - II aus Fig. 1;

Fig. 3 einen Abschnitt des Beleuchtungsmoduls mit OCT-System;

Fig. 4 eine Intensitätsverteilung des aus dem Lichtleiter des OCT-Systems im Operationsmikroskop austretenden OCT-Abtastlichtstrahls; und

Fig. 5 eine Intensitätsverteilung des OCT-Abtastlichtstrahls in der OCT-Abtastebene im Objektbereich des Operationsmikroskops.

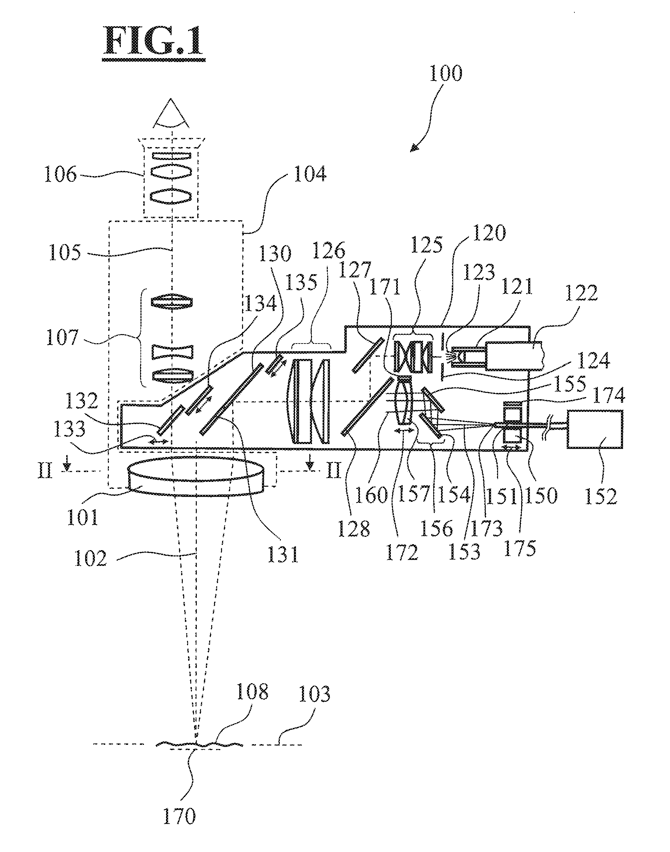

Das Operationsmikroskop 100 in Fig. 1 hat ein Mikroskop-Hauptobjektiv 101 mit einer optischen Achse 102 sowie eines Fokusebene 103, das in einem Operationsmikroskop-Grundkörper 104 aufgenommen ist. Das Mikroskop-Hauptobjektiv 101 wird von stereoskopischen Beobachtungsstrahlengängen 105 eines Binokulartubus 106 durchsetzt. Das Operationsmikroskop 100 enthält ein zoombares Vergrößerungssystem 107.

Zur Beleuchtung des Objektbereichs 108 hat das Operationsmikroskop 100 als Beleuchtungseinrichtung ein Beleuchtungsmodul 120. Dieses Beleuchtungsmodul 120 enthält eine Aufnahme 121 für einen ersten Lichtleiter 122, der Beleuchtungslicht 123 von einer nicht weiter dargestellten Lichtquelle bereitstellt. Mit dem aus dem ersten Lichtleiter 122 austretenden Beleuchtungslicht 123 wird eine verstellbare Leuchtfeldblende 124 ausgeleuchtet. In dem Beleuchtungsmodul 120 ist eine Beleuchtungsoptik angeordnet. Die Beleuchtungsoptik umfasst eine erste Linsengruppe 125, eine zweite Linsengruppe 126, sowie vier Spiegelelemente 127, 128, 129 und 130.

Das Spiegelelement 127 lenkt das aus der ersten Linsengruppe 125 austretende Beleuchtungslicht zu dem Spiegelelement 128, von wo es zu der zweiten Linsengruppe 126 gelangt. Die erste Linsengruppe 125 und die zweiten Linsengruppe 126 bilden die Leuchtfeldblende 124 nach unendlich ab. Somit tritt aus der zweiten Linsengruppe 126 Beleuchtungslicht 123 mit parallelem Strahlengang. Die optische Achse 120 des Beleuchtungsstrahlengangs verläuft austrittsseitig der zweiten Linsengruppe 126 senkrecht zur optischen Achse 102 des Mikroskop-Hauptobjektivs 101. Es wird auf einen Beleuchtungsspiegel 130 geführt, der eine Durchbrechung 131 aufweist. Der Beleuchtungsspiegel 130 lenkt das Beleuchtungslicht parallel zur optischen Achse 102 des Mikroskop-Hauptobjektivs 101 zum Objektbereich 108.

Das Beleuchtungsmodul 120 umfasst weiter einen Beleuchtungsspiegel 132 dem Beleuchtungslicht zugeführt wird, das durch die Durchbrechung 131 in den Beleuchtungsspiegel 130 gelangt.

Der Beleuchtungsspiegel 132 kann entsprechend dem Doppelpfeil 133 senkrecht zur optischen Achse 102 des Mikroskop-Hauptobjektivs 101 verstellt werden. Dies ermöglicht, den Einfallswinkel für Beleuchtungslicht im Objektbereich 108 einzustellen.

Zur Variation der Intensität des im Objektbereich geführten Beleuchtungslichts sind den Beleuchtungsspiegeln 130, 132 verstellbare Blenden 134, 135 zugeordnet. Durch Verstellen der Blenden 134, 135 kann die Intensität des durch das Mikroskop-Hauptobjektiv 101 geführten Beleuchtungslichts variiert werden.

Das Beleuchtungsmodul 120 weist eine Aufnahme 150 für einen zweiten Lichtleiter 151 auf, der mit einem OCT-System 152 verbunden ist.

Das OCT-System 152 ermöglicht zur Untersuchung des Objektbereichs 108 die Aufnahme von OCT-Bildern. Es umfasst eine Einheit für die Erzeugung und Analyse eines OCT-Abtaststrahlengangs 153.

Das OCT-System 152 ist vorzugsweise in eine nicht weiter dargestellte Stativkonsole des Operationsmikroskops integriert. Grundsätzlich könnte es aber auch in dem Beleuchtungsmodul 120 aufgenommen werden.

Der aus dem Lichtleiter 151 austretende Abtaststrahlengang 153 wird auf einen ersten Scanspiegel 154 und einen zweiten Scanspiegel 155 einer OCT-Scaneinheit 156 geführt. Er durchtritt nach der OCT-Scaneinheit 156 eine Sammellinse 157.

Das Strahlenbündel 160 aus der OCT-Scaneinheit 156 wird zu dem Spiegelelement 128 geführt. Das Spiegelelement 128 wirkt als Teilerspiegel. Es reflektiert das aus dem Lichtleiter 122 austretende Beleuchtungslicht nahezu vollständig, ist dabei jedoch für den OCT-Abtaststrahlengang durchlässig. Damit wird der OCT-Abtaststrahlengang 153 dem Beleuchtungslicht 123 überlagert. Das Spiegelelement 128 ist im Beleuchtungsmodul 120 als Spiegelelement mit Planplatten ausgeführt. Es könnte aber auch als Teilerwürfel ausgebildet werden.

Das Licht des OCT-Abtaststrahlengangs 153 wird über die Beleuchtungsspiegel 130, 132 mit dem Beleuchtungslicht zum Objektbereich 108 geführt. Die Sammellinse 157, die zweite Linsengruppe 126 der Beleuchtungsoptik im Beleuchtungsmodul 120 und das Mikroskop-Hauptobjektiv 101 bündeln den OCT-Abtaststrahlengang 153 in einer OCT-Abtastebene 170. Die OCT-Abtastebene 170 ist die Ebene der durch die optischen Elemente im OCT-Abtaststrahlengang mit OCT-Scaneinheit 156, Sammellinse 157, Spiegelelement 128, Spiegelelement 130 sowie Spiegelelement 132 und Mikroskop-Hauptobjektiv 101 festgelegten geometrischen Abbildung des Austrittsendes von Lichtleiter 173 in den Objektbereich 108. D.h., das entsprechende geometrische Bild des Lichtleiter-Austrittsendes liegt in der OCT-Abtastebene 170. Zur Einstellung der OCT-Abtastebene ist einerseits die Sammellinse 152 mittels eines Verstellantriebs 171 entsprechend dem Doppelpfeil 172 bewegbar gehalten. Andererseits kann hierzu das Austrittsende 173 des Lichtleiters 151 für den OCT-Abtaststrahlengang mittels einer Verstellvorrichtung 174 entsprechend dem Doppelpfeil 175 verlagert werden. Bei einem Verstellen der OCT-Abtastebene 170 wird der Referenzstrahlengang im OCT-System soweit erforderlich nachjustiert.

Das in den OCT-Abtaststrahlengang zurückgestreute Licht gelangt über das Mikroskop-Hauptobjektiv 101, die Spiegelelemente 132, 130, die Linsengruppe 126 und das Spiegelelement 128 zurück in das OCT-System 152. Dort wird das aus dem Objektbereich zurückgestreute OCT-Abtastlicht mit OCT-Strahlung aus einem Referenzstrahlengang interferiert. Das Interferenzsignal wird mittels eines Detektors erfasst und durch eine Rechnereinheit ausgewertet, die aus diesem Signal eine optische Weglängendifferenz zwischen Streuzentren für OCT-Licht im Objektbereich und der Weglänge von Licht im Referenzzweig bestimmt.

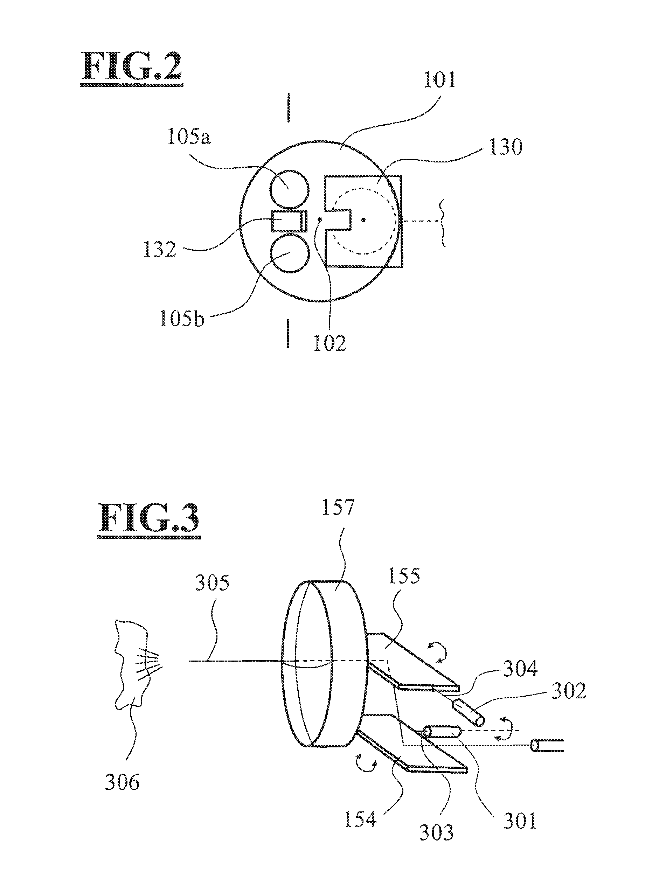

Fig. 2 ist ein Schnitt entlang der Linie II - II aus Fig. 1. Diese Figur zeigt die Beleuchtungsspiegel 130, 132 und erläutert den Verlauf der stereoskopischen Beobachtungsstrahlengänge 105 im Operationsmikroskop 100 aus Fig. 1. Das Mikroskop-Hauptobjektiv 101 wird von zwei stereoskopischen Teilstrahlengängen 105a, 105b durchsetzt. Die optische Achse 102 des Mikroskop-Hauptobjektivs 101 liegt in dessen Zentrum.

Fig. 3 zeigt die OCT-Scaneinheit 156 des Operationsmikroskops 100 aus Fig. 1. Der erste Scanspiegel 154 und der zweite Scanspiegel 155 sind mittels Stellantrieben 301, 302 um zwei zueinander senkrecht verlaufende Achsen 303, 304 drehbeweglich angeordnet. Dies ermöglicht, den OCT-Abtaststrahlengang 305 über eine Ebene 306 zu scannen.

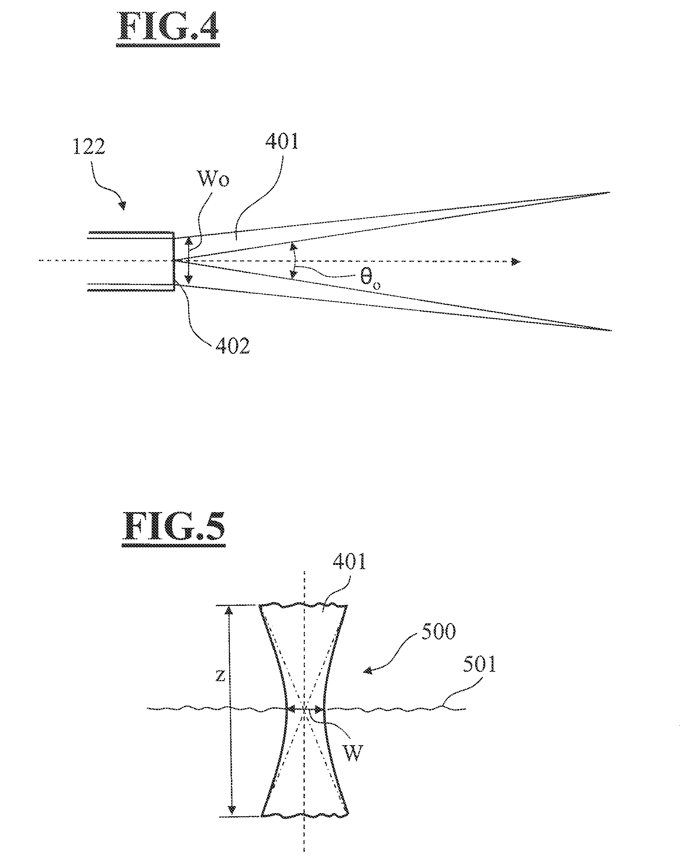

Fig. 4 zeigt den Frontabschnitt 402 des Lichtleiters 151 aus Fig. 1. Der Lichtleiter 151 wirkt für Licht der Wellenlänge $\lambda$ = 1310nm als Monomodenfaser. Der Durchmesser d des Faserkerns des Lichtleiters 122 genügt der Beziehung

$$\frac{d}{2} < 2.4 \, \frac{\lambda}{2\pi \, NA},$$

wobei NA die numerische Apertur der Frontfläche des Lichtleiters ist. Vorzugsweise liegt der Durchmesser d des Faserkerns des Lichtleiters 122 im Bereich 5$\mu$m < d < 10$\mu$m. In diesem Parameterbereich führt der Lichtleiter 122 das Licht mit gaussförmigen Wellenmoden. Aus dem Lichtleiter 151 tritt der OCT-Abtastlichtstrahl 401 mit einem näherungsweise gaussförmigen Strahlungsprofil aus, das durch einen Taillenparamter Wo und einem Öffnungsparameter $\theta_0$ charakterisiert ist, wobei gilt:

$$\theta_0 = \frac{2\lambda}{\pi W_0}$$

Für einen Faserkerndurchmesser von $d_0$ = 10$\mu$m, einen Wellenlänge $\lambda_0$ = 1310 nm ergibt sich damit als Maß für die Strahldivergenz ein Öffnungswinkel von $\theta_0 \approx$ 0,0827 rad.

Die Frontfläche 402 des Lichtleiters 151 wird über die Scanspiegel 154 und 155 im Operationsmikroskop 100 aus Fig. 1, die Sammellinse 157, das Spiegelelement 128, die zweite Linsengruppe 126, das Spiegelelement 130 und das Spiegelelement 132 durch das Mikroskop-Hauptobjektiv 101 auf den Objektbereich 107 in die OCT-Abtastebene 170 abgebildet.

Die Fig. 5 zeigt den Verlauf der Intensitätsverteilung des OCT-Abtastlichtstrahls 401 senkrecht zur OCT-Abtastebene 501. In der OCT-Abtastebene 501 hat die Intensitätsverteilung der OCT-Abtaststrahlung eine kleinste Einschnürung. Außerhalb der OCT-Abtastebene nimmt der Durchmesser des OCT-Abtaststrahlengangs zu. Da der OCT-Abtastlichtstrahl 401 aus dem Lichtleiter 151 aus Fig. 4 mit einem näherungsweise gaussförmigen Strahlungsprofil austritt, bewirken die Sammellinse 157 und das Mikroskop-Hauptobjektiv 101 für den OCT-Abtastlichtstrahl im Bereich der OCT-Abtastebene 170 ein sogenanntes Gaussbündel 500 des OCT-Abtastlichtstrahls 401. Dieses Gaussbündel 500 ist durch den konfokalen Parameter z als Maß für die longitudinale Ausdehnung der Taille des Gaussbündels, sowie den Taillenparameter W als Maß für den Durchmesser der kleinsten Einschnürung 502 des OCT-Abtastlichtstrahls 401, d.h. für den Durchmesser von dessen Taille charakterisiert, wobei gilt:

$$z = 2 \, \frac{W^2 \pi}{\lambda},$$

dabei ist $\lambda$ die Wellenlänge des OCT-Abtastlichtstrahls. Zwischen dem Taillenparametern W des Gaussbündels 500 und dem Taillenparameter Wo des in Fig. 4 gezeigten, aus dem Lichtleiter 151 austretenden Abtastlichtstrahls 401 gilt die folgende Beziehung:

$$W = \beta \, W_0,$$

wobei $\beta$ der Vergrößerungs- bzw. Verkleinerungsparameter der oben erwähnten geometrischen Abbildung des Austrittsendes von Lichtleiter 151 aus Fig. 1 in der OCT-Abtastebene ist. $\beta$ ist mit der Brennweite $f_1$ der Sammellinse 157 aus Fig. 1 und der Brennweite $f_2$ des Mikroskop-Hauptobjektivs über die folgende Beziehung verknüpft:

$$\frac{f_2}{f_1} = \beta$$

Die Größe von Strukturen, die sich mit dem OCT-Abtastlichtstrahl 401 auflösen lassen, ist durch dessen Durchmesser in der OCT-Abtastebene 170, d.h. durch den Taillenparameter W bestimmt. Erfordert beispielsweise eine Applikation eine Lateralauflösung des OCT-Systems im Operationsmikroskop von ca. $40\mu m$, muss nach dem Nyquist-Theoren der Querschnitt des OCT-Abtastlichtstrahls 401 auf der Oberfläche ca. $20\mu m$ betragen. Bei gegebener Wellenlänge $\lambda$ für den OCT-Abtastlichtstrahl 153 aus Fig. 1 müssen daher für eine gewünschte Auflösung des OCT-Systems 152 die Vergrößerung der optischen Abbildung im OCT-Strahlengang und der Durchmesser des Faserkerns im Lichtleiter 151 geeignet gewählt werden.

Der konfokale Parameter z als Maß für die longitudinale Ausdehnung der Taille des Gaussbündels bestimmt den axialen Tiefenbereich, aus dem in dem OCT-Abtaststrahlengang 153 aus Fig. 1 zurückgestreutes Licht detektiert werden kann: Je kleiner der konfokale Parameter z, desto größer ist der Verlust des OCT-Systems an lateraler Auflösung bei Entfernung eines mit OCT-Abtaststrahlung abgetasteten Objekts von der OCT-Abtastebene 170, da sich der Ort von Streuzentren nur innerhalb des durch den Taillenparamter W und den konfokalen Parameter z definierten "Trichter" lokalisieren lässt.

Nachdem die axiale Auflösung eines OCT-Systems einerseits durch die Kohärenzlänge $l_c$ des Lichts der im OCT-System eingesetzten Lichtquelle begrenzt ist, andererseits die laterale Auflösung des OCT-Systems abnimmt, wenn dessen Tiefenhub die mit dem konfokalen Parameter z gegebene Ausdehnung übersteigt, ist das Einstellen des konfokalen Parameters z auf den Tiefenhub des OCT-Systems günstig.

Für eine bestimmte Wellenlänge $\lambda$ des OCT-Abtastlichtstrahls 401 ergibt sich dann die mögliche laterale Auflösung des OCT-Systems aus Fig. 1, da die Wellenlänge $\lambda$ und der konfokale Parameter z den Taillenparameter W festlegen. Die Optikeinheiten im OCT-Abtaststrahlengang 153 aus Fig. 1 und die Bemassung des Faserkerns von Lichtleiter 151 sind dann so zu wählen, dass sich der betreffende Taillenparameter W ergibt.

Das Operationsmikroskop 100 ist so ausgelegt, dass die Fokusebene 170 des Mikroskop-Hauptobjektivs 101 für den sichtbaren Spektralbereich und OCT-Abtastebene 160 zusammenfällt. Dann liegt die in Fig. 5 gezeigte Taille 502 des OCT-Abtastlichstrahls in der Fokusebene des Operationsmikroskops.

Alternativ zu dieser Auslegung des Operationsmikroskops kann auch ein Versatz von OCT-Abtastebene und Fokusebene des Operationsmikroskops vorgesehen sein. Vorzugsweise ist dieser Versatz nicht größer als der konfokale Parameter z des OCT-Abtastlichtstrahls im Bereich der OCT-Abtastebene. Dies ermöglicht es beispielsweise einen unmittelbar unter der Fokusebene des Operationsmikroskops liegenden Objektbereich mittels OCT zu visualisieren. Es kann aber auch sinnvoll sein, für bestimmte Anwendungen einen definierten Versatz vorzusehen, der den konfokalen Parameter übersteigt, etwa um mit dem Operationsmikroskop die Vorderseite der Cornea eines Patientenauges untersuchen zu können und gleichzeitig mittels des OCT-Systems die Rückseite der Cornea des Patientenauges oder dessen Linse zu visualisieren.

Indem die OCT-Abtastebene um den Rayleighparameter z weiter vom Mikroskop-Hauptobjektiv 101 aus Fig. 1 entfernt ist, lässt sich der Tiefenhub für das OCT-System im Objektbereich maximieren.

Eine modifizierte Ausführungsform des anhand von Fig. 1 erläuterten Operationsmikroskops 100 enthält ein fokussierbares Mikroskop-Hauptobjektiv mit einstellbarer Brennweite. Auch diese Maßnahme ermöglicht das Verlagern einer OCT-Abtastebene und das Verändern der geometrischen Abbildung des Lichtleiter-Austrittsendes in die OCT-Abtastebene.

**Patentansprüche**

1.  Operationsmikroskop (100)

    - mit einem Beobachtungsstrahlengang (105) zur Untersuchung eines Objektbereichs (108);
    - mit einem Objektiv (101), das von dem Beobachtungsstrahlengang (105, 105a, 105b) des Operationsmikroskops (100) durchsetzt wird; und
    - mit einem OCT-System (152) zur Untersuchung des Objektbereichs (108);

    wobei das OCT-System (152)

    - einen OCT-Abtaststrahlengang (153) umfasst, der durch das Objektiv (101) geführt ist; und
    - einen Lichtleiter (151) umfasst, der einen Lichtaustrittsabschnitt für den OCT-Abtaststrahlengang (153) aufweist; und
    - eine Kollimationsoptik (157) umfasst, welche den OCT-Abtaststrahlengang mit einer Linsengruppe (126) in einen im wesentlichen parallelen Abtaststrahlengang überführt,

    **dadurch gekennzeichnet, dass**

    - zum Einstellen einer OCT-Abtastebene (170) Mittel zum Bewegen (171, 174) vorgesehen sind, mit denen sich der Lichtaustrittsabschnitt (174) des Lichtleiters (151) und/oder die Kollimationsoptik (157) bewegen lässt.

**2.** Operationsmikroskop nach Anspruch 1, **dadurch gekennzeichnet, dass** die Kollimationsoptik eine Sammellinse (157) umfasst, die mittels eines Verstellantriebs in Richtung des OCT-Abtaststrahlengangs (153) bewegbar ist.

**3.** Operationsmikroskop nach einem der Ansprüche 1 oder 2, **dadurch gekennzeichnet, dass** ein Versatz zwischen der OCT-Abtastebene (170) und einer Fokusebene des Objektivs für den sichtbaren Spektralbereich vorgesehen ist.

**4.** Operationsmikroskop nach Anspruch 3, **dadurch gekennzeichnet, dass** der Versatz nicht größer ist als ein konfokaler Parameter z des OCT-Abtaststrahls im Bereich der OCT-Abtastebene, wobei gilt

$$z = 2\frac{(\beta W_0)^2 \pi}{\lambda}$$

mit $\beta$ : Vergrößerungs- bzw. Verkleinerungsparameter einer geometrischen Abbildung eines Austrittsendes des Lichtleiters 151 in die OCT-Abtastebene; $W_0$ : Taillenparameter eines gaussförmigen Strahlungsprofils am Austrittsende des Lichtleiters (151); und $\lambda$: Wellenlänge des OCT-Abtaststrahls.

**5.** Operationsmikroskop nach einem der Ansprüche 1 bis 4, **dadurch gekennzeichnet, dass** das OCT-System für ein Scannen des OCT-Abtaststrahlengangs einen ersten Scanspiegel (154) umfasst, der um eine erste Drehachse (303) bewegt werden kann.

**6.** Operationsmikroskop nach Anspruch 5, **dadurch gekennzeichnet, dass** ein zweiter Scanspiegel (155) vorgesehen ist, der um eine zweite Drehachse (304) bewegt werden kann, wobei die erste Drehachse (303) und die zweite Drehachse (304) seitlich versetzt in einem rechten Winkel zueinander stehen.

**7.** Operationsmikroskop nach einem der Ansprüche 1 bis 6, **dadurch gekennzeichnet, dass** ein Durchmesser (d) eines Faserkerns des Lichtleiters (151) im Bereich $5\mu$m < d < 10$\mu$m liegt.

**8.** Operationsmikroskop nach einem der Ansprüche 1 bis 7, **dadurch gekennzeichnet, dass** das OCT-System (152) einen Referenzstrahlengang umfasst, und dass der Referenzstrahlengang bei einer Verstellung einer OCT-Abtastebene (170) nachjustierbar ist.

# FIG.1

## FIG.2

## FIG.3

# FIG.4

# FIG.5

| Europäisches Patentamt European Patent Office Office européen des brevets | **EUROPÄISCHER TEILRECHERCHENBERICHT** nach Regel 62a und/oder 63 des Europäischen Patent- übereinkommens. Dieser Bericht gilt für das weitere Verfahren als europäischer Recherchenbericht. | **Nummer der Anmeldung** EP 10 15 5318 |

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (IPC) |
|---|---|---|---|
| A,D | EP 0 815 801 A (ZEISS CARL [DE]; ZEISS STIFTUNG [DE]) 7. Januar 1998 (1998-01-07) * Seite 3, Zeile 53 - Seite 5, Zeile 1 * * Abbildungen 1-3 * ----- | 1-8 | INV. G02B21/06 A61B19/00 G02B21/00 A61B5/00 G01B9/02 |
| A | EP 0 941 692 A (SCHWIND GMBH & CO KG HERBERT [DE] SCHWIND EYE TECH SOLUTIONS GMB [DE]) 15. September 1999 (1999-09-15) * Absätze [0010] - [0017], [0027] * * Abbildung 1 * ----- | 1-8 | |
| A | WO 03/070090 A (ZEISS CARL MEDITEC AG [DE]) 28. August 2003 (2003-08-28) * Absätze [0014] - [0016] * * Abbildung 1 * ----- | 1-8 | |

| | RECHERCHIERTE SACHGEBIETE (IPC) |
|---|---|
| | G02B A61B G01B |

## UNVOLLSTÄNDIGE RECHERCHE

Die Recherchenabteilung ist der Auffassung, daß ein oder mehrere Ansprüche, den Vorschriften des EPÜ nicht entspricht bzw. entsprechen, so daß nur eine Teilrecherche (R.62a, 63) durchgeführt wurde.

Vollständig recherchierte Patentansprüche:

Unvollständig recherchierte Patentansprüche:

Nicht recherchierte Patentansprüche:

Grund für die Beschränkung der Recherche:

Siehe Ergänzungsblatt C

| Recherchenort | Abschlußdatum der Recherche | Prüfer |
|---|---|---|
| München | 14. Juli 2010 | Willig, Hendrik |

| KATEGORIE DER GENANNTEN DOKUMENTE | |
|---|---|
| X : von besonderer Bedeutung allein betrachtet Y : von besonderer Bedeutung in Verbindung mit einer anderen Veröffentlichung derselben Kategorie A : technologischer Hintergrund O : nichtschriftliche Offenbarung P : Zwischenliteratur | T : der Erfindung zugrunde liegende Theorien oder Grundsätze E : älteres Patentdokument, das jedoch erst am oder nach dem Anmeldedatum veröffentlicht worden ist D : in der Anmeldung angeführtes Dokument L : aus anderen Gründen angeführtes Dokument ................................................................. & : Mitglied der gleichen Patentfamilie, übereinstimmendes Dokument |

EPO FORM 1503 03.82 (P04E09)

Europäisches
Patentamt

European
Patent Office

Office européen
des brevets

**UNVOLLSTÄNDIGE RECHERCHE
ERGÄNZUNGSBLATT C**

Nummer der Anmeldung

EP 10 15 5318

Vollständig recherchierbare Ansprüche:
       -

Unvollständig recherchierte Ansprüche:
       1-8

Grund für die Beschränkung der Recherche:

Die Anmeldung erfüllt aus den folgenden Gründen nicht die Erfordernisse von Art. 76(1) EPÜ.

Anspruch 1 beansprucht ein Operationsmikroskop mit Merkmalen der Ansprüche 1 und 8-10 der Stammanmeldung (frühere europäische Patentanmeldung 07 019 793.4) in ihrer ursprünglichen Fassung. Anspruch 1 enthält jedoch nicht sämtliche Merkmale des Anspruchs 1 der Stammanmeldung. Es wurden aus Anspruch 1 der Stammanmeldung die Merkmale weggelassen,
(a) dass das Operationsmikroskop eine Beleuchtungseinrichtung, die eine Beleuchtungsoptik umfasst, welche eine von einer Lichtquelle ausgeleuchtete Leuchtfeldblende zu einem parallelen Beleuchtungsstrahlengang nach unendlich abbildet, aufweist,
(b) dass die Beleuchtungsoptik eine erste Linsengruppe und eine zweite Linsengruppe aufweist, und
(c) dass im Beleuchtungsstrahlengang zwischen der ersten Linsengruppe und der zweiten Linsengruppe ein Einkoppelelement vorgesehen ist, das den OCT-Abtaststrahlengang in den Beleuchtungsstrahlengang einkoppelt.
Außerdem wurde aus Anspruch 1 der Stammanmeldung das Merkmal,
(d) dass das Objektiv im parallelen Beleuchtungsstrahlengang angeordnet ist, um die Leuchtfeldblende auf den Objektbereich abzubilden,
durch das Merkmal,
(e) dass das Objektiv von dem Beobachtungsstrahlengang des Operationsmikroskops durchsetzt wird,
ersetzt.

In der ursprünglichen Offenbarung der Stammanmeldung gibt es keinerlei Basis für das Weglassen bzw. die Ersetzung der genannten Merkmale. Diese Merkmale stellen für die in der Stammanmeldung ursprünglich offenbarten Erfindung erfindungswesentliche Merkmale dar, denn durch sie wird die auf S. 3, Abs. 3 der Stammanmeldung genannte Aufgabe gelöst (vgl. die in den Richtlinien C-VI, 5.3.10 im Zusammenhang mit Art. 123(2) EPÜ genannten Kriterien, die analog auf Art. 76(1) Anwendung finden). Außerdem besteht zwischen der Kollimationsoptik des Anspruchs 9 der ursprünglichen Stammanmeldung und der Beleuchtungseinrichtung des Anspruchs 1 der ursprünglichen Stammanmeldung ein enger funktioneller Zusammenhang. Anspruch 9 der ursprünglichen Stammanmeldung definiert nämlich dass die Kollimationsoptik den OCT-Abtaststrahlengang mit der zweiten Linsengruppe der Beleuchtungsoptik in einen im wesentlichen parallelen Abtaststrahlengang überführt.
Die aus Anspruch 1 der Stammanmeldung weggelassenen Merkmale werden auch in keinem der abhängigen Ansprüche 2-8 eingeführt.
Demzufolge geht der gesamte beanspruchte Gegenstand über den Inhalt der Stammanmeldung in der ursprünglich eingereichten Fassung hinaus.

Die Recherche wurde unter der Annahme durchgeführt, dass die oben

Europäisches
Patentamt

European
Patent Office

Office européen
des brevets

**UNVOLLSTÄNDIGE RECHERCHE
ERGÄNZUNGSBLATT C**

genannten Merkmale (a), (b), (c) und (d) im Anspruch 1 enthalten sind.

EP 2 221 653 A1

**ANHANG ZUM EUROPÄISCHEN RECHERCHENBERICHT
ÜBER DIE EUROPÄISCHE PATENTANMELDUNG NR.**

EP 10 15 5318

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

14-07-2010

| Im Recherchenbericht angeführtes Patentdokument | Datum der Veröffentlichung | Mitglied(er) der Patentfamilie | Datum der Veröffentlichung |
|---|---|---|---|
| EP 0815801 A | 07-01-1998 | DE 69724193 D1<br>DE 69724193 T2<br>JP 4091143 B2<br>JP 10071158 A<br>US 5795295 A | 25-09-2003<br>17-06-2004<br>28-05-2008<br>17-03-1998<br>18-08-1998 |
| EP 0941692 A | 15-09-1999 | AT 223676 T<br>ES 2183447 T3<br>US 6095648 A | 15-09-2002<br>16-03-2003<br>01-08-2000 |
| WO 03070090 A | 28-08-2003 | CA 2475046 A1<br>EP 1480552 A2<br>JP 4262603 B2<br>JP 2005517483 T<br>US 2003160942 A1<br>US 2003160943 A1 | 28-08-2003<br>01-12-2004<br>13-05-2009<br>16-06-2005<br>28-08-2003<br>28-08-2003 |

Für nähere Einzelheiten zu diesem Anhang : siehe Amtsblatt des Europäischen Patentamts, Nr.12/82

14

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0815801 A
- US 5321501 A

- WO 200610544 A1